# EUROPEAN PATENT APPLICATION

(11) **EP 1 484 012 A1**
(43) Date of publication of application: **08.12.2004**
(21) Application number: 03705320.4
(22) Date of filing: 19.02.2003
(51) Int. Cl.: A61B 5/15, A61N 1/30

(54) **ELECTRODE STRUCTURAL BODY**

(30) Priority: 22.02.2002 JP 2002047029
(71) Applicant: HISAMITSU PHARMACEUTICAL CO. INC., Tosu-shi, Saga-ken 841-0017 (JP); Kyodo Printing Co., Ltd., Tokyo 112-8501 (JP)
(72) Inventor: MORI, K., c/o Hisamitsu Pharmaceutical Co., Inc., Tsukuba-shi, Ibaraki 305-0856 (JP); MAEDA, Hi, c/o Hisamitsu Pharmaceutical Co., Inc., Tsukuba-shi, Ibaraki 305--0856 (JP); NISHI, Y., c/o Hisamitsu Pharmaceutical Co., Inc., Tsukuba-shi, Ibaraki 305-0856 (JP); ARIMOTO, T., c/o Hisamitsu Pharmaceutical Co., Inc, Tsukuba-shi, Ibaraki 305-0856 (JP); HIGO, N., c/o Hisamitsu Pharmaceutical Co., Inc., Tsukuba-shi, Ibaraki 305-0856 (JP); SATO, S. c/o Hisamitsu Pharmaceutical Co., Inc., Tsukuba-shi, Ibaraki 305-0856 (JP); OGAWA, T., c/o Kyodo Printing Co., Ltd., Bunkyo-ku, Tokyo 112-8501 (JP); TAKAHASHI, S, c/o Kyodo Printing Co., Ltd., Bunkyo-ku, Tokyo 112-8501 (JP); FUCHITA, Y, c/o Kyodo Printing Co., Ltd., Bunkyo-ku, Tokyo 112-8501 (JP)
(74) Representative: Westendorp, Michael, Dr.
(86) International application number: PCT/JP2003/001781
(87) International publication number: WO 2003/070100

(57) **Abstract**

There is provided an electrode structure in which cracks are hardly generated in the bending processed portions, in particular, in the electrode layer and the insulating layer. The electrode structure comprises a support (3) having bending processed portions (4), an electrode layer (1) formed on the support so as to pass over the bending processed portions (4), and an insulating layer (2) formed on the electrode layer (1) passing over the bending processed portions (4). The glass transition temperature of the dielectric material forming the insulating layer (2) is made to be 25°C or below. The electrode layer (1) includes at least one material belonging to the group consisting of silver, silver chloride and carbon, and in particular, a terminal portion (5) of the electrode layer (1) passing over the bending processed portions (4) is formed of a paste containing carbon as the main component.

## Description

### Technical Field

The present invention relates to an electrode structure suitable for use as an electrode for application to living body used in the medical care fields including medical treatment and diagnosis. Such an electrode structure is applied to a device for delivering a physiologically active substance into a living body by utilizing electric energy and a device for extracting a diagnostic substance from the interior of a living body to the outside by utilizing electric energy.

### Background Art

Iontophoresis (for example, Acta Dermatol Venererol, Vol. 64, p. 93, 1984) and electroporation (for example, National Publication of International Patent Application No. 3-502416, andProc. Natl. Acad. Sci. USA, Vol. 90, pp. 10504-10508, 1933) are methods for delivering drugs across the skin and mucosa by use of electric energy. Additionally, there is a method in which diagnostic substances are extracted from a living body on the basis of the same principle and the condition of a disease is thereby scrutinized (for example, Nature Medicine, Vol. 1, pp. 1198-1120, 1955). For the purpose of implementing these methods, devices for delivering physiologically active substances and devices for extracting diagnostic substances from living bodies all need electrode structures including electrodes.

Generally, an electrode structure is provided with a depression in which a gel made of a polymer or the like containing an electrolyte is placed. For example, an electrode structure is fabricated as follows: at the beginning, a flat film is coated with an electrode layer with a terminal attached; additionally an insulating layer is provided on the portion other than the portion functioning as an electrode; and by molding the flat film coated with the electrode layer and the insulating layer, a cup like electrode structure having a depression is fabricated.

However, there has so far been a problem such that when a cup like electrode structure is fabricated, cracks are generated in the bending processed portions, in particular, in the electrode layer and the insulating layer. The generation of these cracks must be avoided because there is a fear such that the exposure of the electrode causes electric leak.

Accordingly, an object of the present invention is to provide an electrode structure in which cracks are hardly generated in the bending processed portions, in particular, in the electrode layer and the insulating layer.

### Disclosure of the Invention

The above described object can be achieved by an electrode structure including a support having bending processed portions, an electrode layer formed on the support so as to pass over the bending processed portions, and an insulating layer formed on the electrode layer passing over the bending processed portions, the glass transition temperature of at least one dielectric material of the dielectric materials forming the insulating layer being 25°C or below. In this connection, the thickness of the insulating layer can be made to be 0.5 µm to 100 µm. Additionally, the support can be formed with a polyethylene terephthalate film or an insulating base formed with an aluminum foil coated or laminated with an insulating film. The electrode layer can include at least one material belonging to the group consisting of silver, silver chloride and carbon. The portions of the electrode layer passing over the bending processed portions can be formed of a paste which contains carbon as the main component. The interior angles and the conjugate angles of the bending processed portions can be made to be 90 degrees to 270 degrees.

Additionally, a method for manufacturing an electrode structure involved in the present invention comprises the steps of forming an electrode layer having a terminal portion on a support, forming an insulating layer, which includes a dielectric material having a glass transition temperature of 25°C or below, on the terminal portion of the electrode layer, and performing a bending process of specific portions of the support including the insulating layer. In this connection, it is preferable to form the insulating layer by the screen printing.

By forming as described above, there can be obtained an electrode structure in which cracks are hardly generated in the bending processed portions, inparticular, intheelectrode layer and the insulating layer.

### Brief Description of the Drawings

Figure 1 is a diagram illustrating an example of a configuration of an electrode structure prior to molding, according to the present invention, (a) being a plane view and (b) being a sectional view along the X-X' segment in (a);
Figure 2 is a sectional view illustrating an example of a configuration of an electrode structure after the molding, according to the present invention; and
Figure 3 is a sectional view illustrating an example of a support used in the present invention.

### Best Mode for Carrying Out the Invention

Now, description will be made below on the embodiments of the present invention.

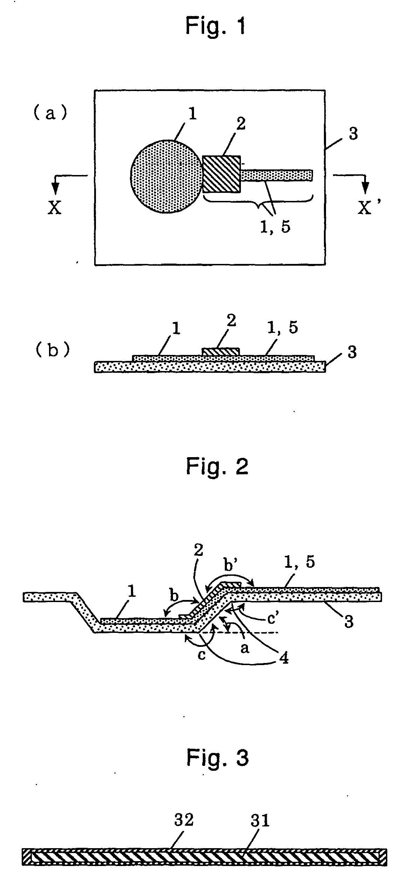

Figure 1 is a diagram illustrating an example of a configuration of an electrode structure prior to molding, according to the present invention, (a) being a plane view and (b) being a sectional view along the X-X' segment in (a). As shown in the figure, an electrode layer 1 having a terminal portion 5 is formed on a support 3, and moreover, an insulating layer 2 is formed on the support 3 including the terminal portion 5 of the electrode layer 1. The insulating layer 2 is a member formed by coating of a paste of a dielectric material, for example, with the aid of the screen printing.

Figure 2 is a sectional view illustrating an example of a configuration of an electrode structure after the molding, according to the present invention. As shown in the figure, the part of the support 3, including the insulating layer 2, is processed into a bent form. Accordingly, a depression is formed in the support 3. Therefore, the support 3 possesses the bending processed portions 4 formed at the time of molding. The terminal portion 5 of the electrode layer 1 is extended on the support 3 to the outside so as to pass over the bending processed portions 4. The insulating layer 2 is positioned on the terminal portion 5 of the electrode layer 1 passing over the bending processed portions 4.

The present inventors discovered that the main causes for generating cracks in the electrode layer and the insulating layer at the time of molding are the following two facts:
(1) The insulating layer is not extended at the time of molding.
(2) The electrode layer does not comply with the extension of the film at the time of molding.

The present invention has been achieved on the basis of the investigation of these causes.

First of all, various dielectric materials to be used for the insulating layer were tested. Consequently, it has been found that when there is used a dielectric material having a glass transition temperature of 25°C or below, no cracks are generated at the time of molding, and moreover, when there is used a dielectric material having a glass transition temperature of 0°C or below, in particular, -20°C or below, no cracks are generated even for the case where the angles of the bending processed portions are large (the conjugate angles are large).

The thickness of the insulating layer formed of a dielectric material is made to be 0. 5 µm to 100 µm, preferably 2 µm to 50 µm. With this thickness, the insulating layer can flexibly response to the extension while maintaining the insulating property.

Examples of the dielectric materials include polydiene, polyacryl, polymethacryl, acrylamide, polyethylene, polyvinyl ester, polyester, polyurethane, polysiloxane, polyamide (nylon), polyacetal and polypropylene; however, the dielectric materials are not limited to these examples.

As the method for coating the dielectric material, the screen printing method is used. This method is excellent in that the method permits an easy control of the coating thicknes s, and can draw a portion to be printed with an accurate pattern.

In the next place, as for the electrode layer (the electrode and the terminal portion), it is recommended to use a paste containing as the main component at least one of silver, silver chloride and carbon. In particular, because no polarization occurs, it is recommended to use silver as the electrode material for the anode section, while it is recommended to use silver chloride containing silver (silver/silver chloride) as the electrode material for the cathode section. Additionally, the portion of the electrode terminal portion suffering stress at the time of molding a depression, namely, the portions of the electrode terminal portion subjected to bending (the bending processed portions) are liable to be cracked. Particularly for these portions, it is recommended to use a conductive paste containing carbon as the main component. The present inventors discovered that by printing the conductive paste containing carbon as the main component, the electrode terminal portion is made to have high compliance, and in addition to the electrode terminal portion (carbon layer), even the insulating layer laminated on the electrode terminal portion comes to be hardly cracked. In this case, the coating thickness of the carbon layer is made to be 0.5 µm to 100 µm, preferably 1 µm to 75 µm, further preferably 2 µm to 25 µm. With this thickness, the electrode terminal portion is excellent in conductivity and compliance.

The support is formed with an insulating base, the electrode and the electrode terminal are coated on the support, and moreover, the support is molded into a cup shape having a depression; a drug or an electrolyte gel is to be held in the depression. Therefore, the support is required to be made of a material high in moldability and hardly deformable after molding. As for the support, for example, polyethylene terephthalate film meets this condition, and moreover, this film is an insulator, and accordingly can be preferably used as a support.

Additionally, for the support, there can be used materials based on metals excellent in moldabiltiy such as aluminum. These materials are conductive, and hence cannot be used without modification; it is necessary to apply an insulating coat or laminate to the surface of any of these metal materials. Examples of the insulating coat materials include polydiene, polyacryl, polymethacryl, acrylamide, polyvinyl alcohol, polyethylene, polyvinyl ester, polystyrene, polycarbonate, polyester, polyurethane, polysiloxane, polyamide, polyacetal and polyacrylonitrile; however, the insulating coat materials are not limited to these examples. Examples of the insulating laminate materials include polyester, nylon, polypropylene, polyethylene, cellophane and polyacrylonitrile; however, the insulating laminate materials are not limited to these examples.

For the metal based support, aluminum is preferable because aluminum materials are easily available. Figure 3 is a sectional view illustrating an example of a support used in the present invention. As shown in the figure, the support of the present example is fabricated by coating or laminating an insulating film 32 on a sheet of aluminum 31. The sheet of aluminum 31 is made to be preferably 6 µm to 100 µm in thickness, more preferably 12 µm to 75 µm. The insulated aluminum film thus fabricated is excellent in moldability, and suitable as an insulating base. A dielectric material is needed to be coated directly not only on the electrode terminal portion but also on the support; otherwise, there occur problems involving electric leak and the like. In this connection, polyethylene terephthalate film and the insulated aluminum film can be easily coated with a dielectric material and an electrode paste, and hence are suitable as the support (insulating base).

A bending processed portion means a portion formed by bending a support by 20 degrees or more in relation to the plane. In Figure 2, the angles associated with the respective bending processed portions 4 are represented as follows: an angle by which the bending is carried out is represented by the bending angle a; the angles generated by this bending, as viewed from the insulating layer 2, are represented by the interior angles b and b'; and the angles generated by this bending, as viewed from the side opposite to the insulating layer 2, are represented by the respective conjugate angles c and c'. As far as either the interior angles or the conjugate angles fall within the range from 90 degrees to 270 degrees, cracks are hardly generated in the insulating layer and the electrode layer; however, when these angles are smaller or larger than this range, cracks come to be easily generated.

As described above, for the purpose of providing an excellent electrode structure for use in an electrode for application to living body, the present invention specifies the selection of the dielectric material as the insulating layer; the coating method and the coating thickness of the dielectric material; the material for the electrode layer and the terminal portion thereof and the coating thickness of the material; and the material for the support and the bending angle of the support, and the like.

### (Examples)

### Experimental Example 1

Evaluation was carried out on the generation of cracks in the bending processed portions when the bending processed portions were formed by the compression molding of the supports (insulating bases) coated respectively with dielectric materials respectively having the glass transition temperatures of 85, 67, 45, 40, 35, 25, 5, 1, -20 and -29°C.

### (Example 1)

As shown in Figure 1, on an insulated aluminum film formed as the support 3 by laminating a 38 µm thick polyester film on a 50 µm thick aluminum plate, an about 20 µm thick carbon paste layer was formed as a terminal portion 5 by coating with the aid of the screen printing. As an electrode layer 1, a circular form of 40 µm thick silver paste layer with the silver content of 90%(w/w) was formed by coating so as to partially overlap with the terminal portion 5. After drying, as an insulating layer, a 15 µm thick layer of a dielectric material having a glass transition temperature of 25°C (a polyester based resin with the brand name of Vylon GK150, manufactured by Toyobo Co., Ltd.) was formed by coating with the aid of the screen printing method so as to partially cover the terminal portion 5. The laminate thus obtained was subjected to compression molding, and a depression was thereby formed as shown in Figure 2. The generation of cracks in the bending processed portions 4 at the time of molding was evaluated.

### (Example 2)

An evaluation similar to that in Example 1 was carried out for the dielectric material having a glass transition temperature of 5°C (a polyester based resin with the brand name of Elitel UE3220, manufactured by Unichika Ltd.).

### (Example 3)

An evaluation similar to that in Example 1 was carried out for the dielectric material having a glass transition temperature of 2°C (a polyester based resin with the brand name of Elitel UE3221, manufactured by Unichika Ltd.).

### (Example 4)

An evaluation similar to that in Example 1 was carried out for the dielectric material having a glass transition temperature of -20°C (a polyester based resin with the brand name of Elitel UE3400, manufactured by Unichika Ltd.).

### (Example 5)

An evaluation similar to that in Example 1 was carried out for the dielectric material having a glass transition temperature of -29°C (a polyester based resin with the brand name of Elitel UE3410, manufactured by Unichika Ltd.).

### (Comparative Example 1)

Similarly to Example 1, on an insulated aluminum film (a film formed by laminating PET on an aluminum plate), the silver paste was printed, and thereafter, a 15 µm thick layer of a dielectric material having a glass transition temperature of 85°C (a polyester based resin with the brand name of Elitel UE3690, manufactured by Unichika Ltd.) was formed by coating with the aid of the screen printing. Similarly to Example 1, the laminate thus obtained was subjected to compression molding, and the generation of cracks was evaluated.

### (Comparative Example 2)

An evaluation similar to that in Example 1 was carried out for the dielectric material having a glass transition temperature of 67°C (a polyester based resin with the brand name of Vylon GK200, manufactured by Toyobo Co., Ltd.).

### (Comparative Example 3)

An evaluation similar to that in Example 1 was carried out for the dielectric material having a glass transition temperature of 45°C (a polyester based resin with the brand name of Elitel UE3210, manufactured by Unichika Ltd.).

### (Comparative Example 4)

An evaluation similar to that in Example 1 was carried out for the dielectric material having a glass transition temperature of 40°C (a polyester based resin with the brand name of Elitel UE3240, manufactured by Unichika Ltd.).

### (Comparative Example 5)

An evaluation similar to that in Example 1 was carried out for the dielectric material having a glass transition temperature of 35°C (a polyester based resin with the brand name of Elitel UE3500, manufactured by Unichika Ltd.).

The results of the experimental example 1 are as shown in Table 1.

**Table 1**

| | Tg (°C) | Processing angle (Conjugate angle) | |
|---|---|---|---|
| | | 230° | 250° |
| Comparative example 1 | 85 | 18/18 | 18/18 |
| Comparative example 2 | 67 | 18/18 | 18/18 |
| Comparative example 3 | 45 | 18/18 | 18/18 |
| Comparative example 4 | 40 | 18/18 | 18/18 |
| Comparative example 5 | 35 | 18/18 | 18/18 |
| Example 1 | 25 | 2/18 | 17/18 |
| Example 2 | 5 | 0/18 | 16/18 |
| Example 3 | 2 | 0/18 | 12/18 |
| Example 4 | -20 | 0/18 | 0/18 |
| Example 5 | -29 | 0/18 | 0/18 |
| (Number of cracked specimens/total number) | | | |

As shown in Table 1, in the cases of the processing with a conjugate angle of 230°, for the dielectric materials having a glass transition temperature of 5 to -29°C, no electrode structures exhibited cracks among the total number of 18 electrode structures. Additionally, for the dielectric material having a glass transition temperature of 25°C, cracks were generated in some cases, but the number of such cases was small. Additionally, in the cases of the processing with a conjugate angle of 250°, for the dielectric materials having a glass transition temperature of -20 to -29°C, no electrode structures exhibited cracks, and some cases were observed not to exhibit cracks even for the glass transition temperature of 25°C.

### Experimental Example 2

As for the generation of cracks, the cases where carbon pastes were used for the electrode terminal portion (Examples 1, 2) were compared with the cases where silver pastes were used for the electrode terminal portion (Comparative Examples 6, 7).

### (Comparative Example 6)

As shown in Figure 1, on an insulated aluminum film formed as the support 3 by laminating a 38 µm thick polyester film on a 50 µm thick aluminum plate, an about 20 µm thick silver paste layer with the silver content of 90%(w/w) was formed as a terminal portion 5 by coating with the aid of the screen printing. As an electrode layer 1, a circular form of 40 µm thick layer of this silver paste was formed by coating so as to partially overlap with the terminal portion 5. Afterdrying, as an insulating layer, a 15 µm thick layer of the dielectric material having a glass transition temperature of 25°C (a polyester based resin with the brand name of Vylon GK150, manufactured by Toyobo Co., Ltd.) was formed by coating with the aid of the screen printing so as to partially cover the terminal portion 5. The laminate thus obtained was subjected to compression molding, and a depression was thereby formed as shown in Figure 2. The generation of cracks in the bending processed portions 4 at the time of molding was evaluated.

### (Comparative Example 7)

An evaluation similar to that in Comparative Example 6 was carried out for the dielectric material having a glass transition temperature of 5°C (a polyester based resin with the brand name of Elitel UE3220, manufactured by Unichika Ltd.).

The results of the experimental example 2 are as shown in Table 2.

**Table 2**

| | Presence/absence of carbon | Tg (°C) | Processing angle (Conjugate angle) |
|---|---|---|---|
| | | | 250° |
| Comparative example 6 | Absent | 25 | 18/18 |
| Comparative example 7 | Absent | 5 | 18/18 |
| Example 1 | Present | 25 | 17/18 |
| Example 2 | Present | 5 | 16/18 |
| Number of cracked specimens/total number | | | |

As shown in Table 2, in the cases of the processing with a conjugate angle of 250°, for the electrode structures in which carbon was used for the bending processed portions as in the cases of Examples 1, 2, no cracks were found to be generated in some electrode structures even for the cases of the dielectric materials having a glass transition temperature of 25°C or 5°C; however, for the electrode structures in which no carbon is used as in the cases of Comparative Examples 6, 7, cracks were observed to be generated in all the examples.

### Industrial Applicability

According to the present invention, there can be obtained an electrode structure which is hardly cracked in the bending processed portions, in particular, in the electrode layer and the insulator layer.

## Claims

1. An electrode structure comprising a support having bending processed portions, an electrode layer formed on the support so as to pass over the bending processed portions, and an insulating layer formed on the electrode layer passing over the bending processed portions, wherein the glass transition temperature of at least one dielectric material of the dielectric materials forming the insulating layer is 25°C or below.

2. The electrode structure according to claim 1, wherein the thickness of the insulating layer is 0.5 µm to 100 µm.

3. The electrode structure according to claim 1 or 2, wherein the support is formed with a polyethylene terephthalate film or an insulating base formed with an aluminum foil coated or laminated with an insulating film.

4. The electrode structure according to any one of claims 1 to 3, wherein the electrode layer comprises at least one material belonging to the group consisting of silver, silver chloride and carbon.

5. The electrode structure according to any one of claims 1 to 4, wherein the portions of the electrode layer passing over the bending processed portions are formed of a paste which contains carbon as the main component.

6. The electrode structure according to any one of claims 1 to 5, wherein the interior angles and the conjugate angles of the bending processed portions are 90 degrees to 270 degrees.

7. A method for manufacturing an electrode structure, comprising the steps of:
forming an electrode layer having a terminal portion on a support;
forming an insulating layer, which includes a dielectric material having a glass transition temperature of 25°C orbelow, on the terminal portion of the electrode layer; and
performing a bending process of specific portions of the support including the insulating layer.

8. The method for manufacturing the electrode structure according to claim 7, wherein the insulating layer is formed by a screen printing.
